# EUROPÄISCHE PATENTSCHRIFT

(11) **EP 1 809 293 B1**
(45) Veröffentlichungstag und Bekanntmachung des Hinweises auf die Patenterteilung: **16.03.2016**
(21) Anmeldenummer: 05747843.0
(22) Anmeldetag: 10.05.2005
(51) Int. Cl.: A61K 31/536, A61P 11/06

(54) **AEROSOLFORMULIERUNG FÜR DIE INHALATION VON BETAAGONISTEN**
AEROSOL FORMULATION FOR INHALATION OF BETA AGONISTS
FORMULATION D'AEROSOL POUR L'INHALATION DE BETA-AGONISTES

(30) Priorität: 14.05.2004 DE 102004024452
(43) Veröffentlichungstag der Anmeldung: 25.07.2007
(73) Patentinhaber: BOEHRINGER INGELHEIM INTERNATIONAL GMBH, 55216 Ingelheim (DE); Boehringer Ingelheim Pharma GmbH & Co. KG, 55216 Ingelheim (DE)
(72) Erfinder: AVEN, Michael, 55131 Mainz (DE)
(74) Vertreter: Simon, Elke Anna Maria
(86) Internationale Anmeldenummer: PCT/EP2005/005028
(87) Internationale Veröffentlichungsnummer: WO 2005/110421

(56) Entgegenhaltungen:
- EP-A- 0 073 505
- WO-A-2004/045618
- WO-A-2005/102349
- WO-A-2005/102350
- WO-A-2005/110359
- WO-A-2005/111005
- US-A- 4 460 581

## Beschreibung

Die vorliegende Erfindung betrifft eine treibgasfreie Aerosolformulierung, enthaltend als alleinigen Wirkstoff eine Verbindung der allgemeinen Formel **1** 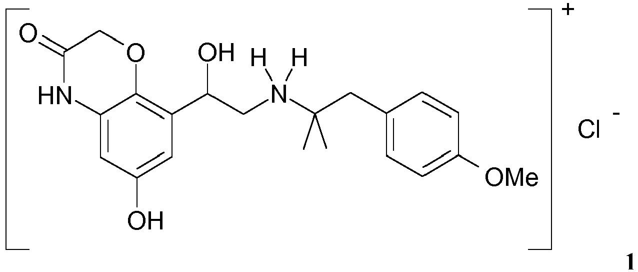 gegebenenfalls in Form Ihrer Tautomere, Enantiomere, Mischungen der Enantiomere, Racemate oder Solvate, wenigstens eine pharmakologisch verträgliche Säure, gegebenenfalls weitere pharmakologisch verträgliche Hilfsstoffe und/oder Komplexbildner sowie als Lösungsmittel Wasser, Ethanol oder ein Gemisch aus Wasser und Ethanol, wobei die Konzentration der Verbindung der Formel **1** bezogen auf den Anteil an pharmakologisch wirksamer freien Base **1'** bei 0,75 bis 200 mg pro 100 ml liegt.

### HINTERGRUND DER ERFINDUNG

Betamimetika (β-adrenerge Substanzen) sind aus dem Stand der Technik bekannt. Beispielsweise sei diesbezüglich auf die Offenbarung der US 4,460,581 verwiesen, die Betamimetika zur Therapie unterschiedlichster Erkrankungen vorschlägt.

Zur medikamentösen Therapie von Erkrankungen ist es häufig wünschenswert, Arzneimittel mit einer längeren Wirkungsdauer bereitzustellen. Hierdurch kann in der Regel gewährleistet werden, dass die zur Erzielung des therapeutischen Effekts erforderliche Konzentration des Wirkstoffs im Organismus über einen längeren Zeitraum gegeben ist, ohne eine allzu häufige, wiederholte Gabe des Arzneimittels durchführen zu müssen. Die Applikation eines Wirkstoffs in längeren zeitlichen Abständen trägt im Übrigen in hohem Maße zum Wohlbefinden des Patienten bei.

Besonders wünschenswert ist die Bereitstellung eines Arzneimittels, welches therapeutisch sinnvoll durch einmalige Applikation pro Tag (Einmalgabe) eingesetzt werden kann. Eine einmal pro Tag erfolgende Anwendung hat den Vorteil, dass der Patient sich relativ schnell an die regelmäßige Einnahme des Medikaments zu bestimmten Tageszeiten gewöhnen kann.

Es ist daher Aufgabe der vorliegenden Erfindung, eine Arzneimittelformulierung für die Inhalation bereitzustellen, die einerseits beispielsweise bei der Therapie von Atemwegserkrankungen einen therapeutischen Nutzen entfaltet und darüber hinaus durch eine längere Wirkdauer gekennzeichnet ist und somit zur Herstellung eines Arzneimittels mit längerer Wirksamkeit Verwendung finden kann.

### DETAILLIERTE BESCHREIBUNG DER ERFINDUNG

Zur Lösung der vorstehend genannten Aufgaben schlägt die vorliegende Erfindung die nachstehende Arzneimittelformulierung vor.

Die erfindungsgemäßen Arzneimittelformulierungen ist eine treibgasfreie Arzneimittelformulierung, enthaltend als alleinigen Wirkstoff eine Verbindung der allgemeinen Formel **1** 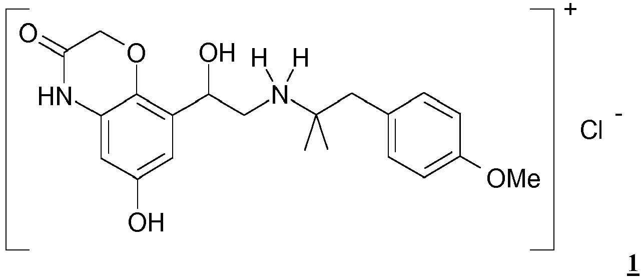 gegebenenfalls in Form Ihrer Tautomere, Enantiomere, Mischungen der Enantiomere, Racemate oder Solvate, wenigstens eine pharmakologisch verträgliche Säure, gegebenenfalls weitere pharmakologisch verträgliche Hilfsstoffe und/oder Komplexbildner sowie als Lösungsmittel Wasser, Ethanol oder ein Gemisch aus Wasser und Ethanol, wobei die Konzentration der Verbindung der Formel **1** bezogen auf den Anteil an pharmakologisch wirksamer freien Base **1'** bei 0,75 bis 200 mg pro 100 ml liegt

Die erfindungsgemäße Arzneimittelformulierung enthält als Lösungsmittel reines Wasser, reines Ethanol oder Mischungen aus Ethanol und Wasser. Werden Ethanol-Wasser-Mischungen verwendet, so liegt der prozentuale Massenanteil von Ethanol in diesen Mischungen bevorzugt im Bereich zwischen 5 und 99 % Ethanol, besonders bevorzugt im Bereich von 10 bis 96 % Ethanol. Eine ganz besonders bevorzugte Arzneimittelformulierung im Sinne der vorliegenden Erfindung enthält als Lösungsmittel reines Wasser, reines Ethanol oder Ethanol-Wasser-Mischungen enthaltend zwischen 50 und 92 %, besonders bevorzugt zwischen 69 und 91% Ethanol. Gegebenenfalls können neben Ethanol und Wasser weitere Co-Solventien eingesetzt werden. Erfindungsgemäß bevorzugt gelangt ein weiteres Lösungsmittel allerdings nicht zum Einsatz.

Die Herstellung der erfindungsgemäßen Verbindung der Formel **1** kann in Analogie zu im Stand der Technik bereits bekannten Vorgehensweisen erfolgen. Geeignete Herstellverfahren sind beispielsweise aus der US 4460581 bekannt, auf die an dieser Stelle vollinhaltlich Bezug genommen wird.

Die Verbindung der Formel **1** kann in der erfindungsgemäßen Arzneimittelformulierung gegebenenfalls in Form ihrer Tautomere enthalten sein. Unter Tautomerie versteht man das Auftreten isomerer Verbindungen, die unter Verschiebung von σ- oder π-Bindungen entstehen und im Gleichgewicht vorliegen können. Beispiele für mögliche tautomere Formen der Verbindungen der Formel **1** sind 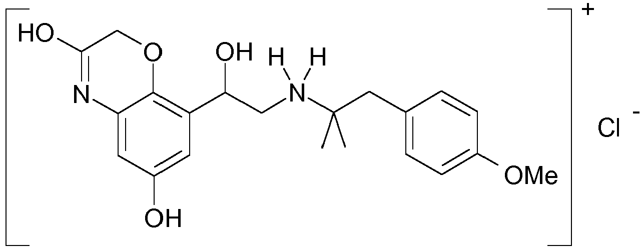 oder auch

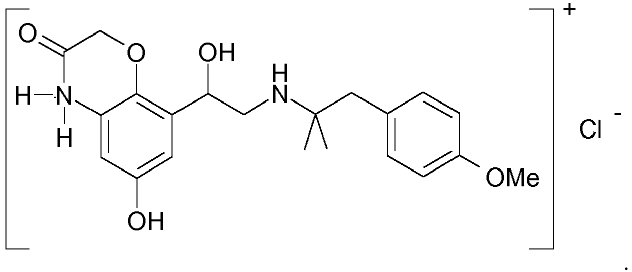

Ein weiterer Aspekt der vorliegenden Erfindung betrifft eine Arzneimittelformulierung, die die vorstehend genannte Verbindung der Formel **1** in Form der einzelnen optischen Isomere, Mischungen der einzelnen Enantiomere oder Racemate enthält. Besonders bevorzugt ist hierbei eine Arzneimittelformulierung, die die vorstehend genannte Verbindung der Formel **1** in Form der enantiomerenreinen Verbindung enthält, wobei hierbei das R-Enantiomer der Verbindung der Formel **1** erfindungsgemäß von herausragender Bedeutung ist. Dieses R-Enantiomere ist durch die Formel ***R-1*** darstellbar

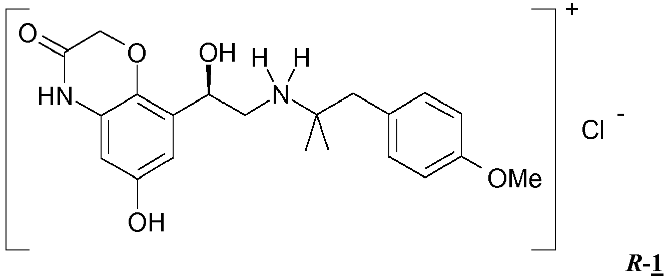

Ein weiterer Aspekt der vorliegenden Erfindung betrifft die Verwendung der erfindungsgemäßen Arzneimittelformulierung zur Herstellung eines Arzneimittels zur Behandlung von Atemwegserkrankungen, die ausgewählt sind aus der Gruppe bestehend aus Obstruktive Lungenerkrankungen unterschiedlicher Genese, Lungenemphyseme unterschiedlicher Genese, Restriktive Lungenerkrankungen, Interstitielle Lungenerkrankungen, Zystische Fibrose, Bronchitiden unterschiedlicher Genese, Bronchiektasen, ARDS (adult respiratory distress syndrom) und alle Formen des Lungenödems.

Bevorzugt ist die vorstehend genannte Verwendung zur Herstellung eines Arzneimittels zur Behandlung von Obstruktive Lungenerkrankungen die ausgewählt sind aus der Gruppe bestehend aus Asthma Bronchiale, pädiatrisches Asthma, schweres Asthma, akuter Asthma-Anfall, chronische Bronchitis und chronisch obstruktive Lungenerkrankung (COPD), wobei die Verwendung zur Herstellung eines Arzneimittels zur Behandlung von Asthma Bronchiale oder COPD erfindungsgemäß besonders bevorzugt ist.

Bevorzugt ist ferner die Verwendung der erfindungsgemäßen Arzneimittelformulierung zur Herstellung eines Arzneimittels zur Behandlung von Lungenemphysemen die ihren Ursprung haben in COPD (chronisch obstruktive pulmonale Erkrankung) oder α1-Proteinase-Inhibitor-Mangel.

Bevorzugt ist ferner die Verwendung der erfindungsgemäßen Arzneimittelformulierung zur Herstellung eines Arzneimittels zur Behandlung von Restriktiven Lungenerkrankungen, die ausgewählt sind aus der Gruppe bestehend aus Allergische Alveolitis, durch berufliche Noxen ausgelöste restriktive Lungenerkrankungen wie Asbestose oder Silikose und Restriktion aufgrund von Lungentumoren, wie beispielsweise Lymphangiosis carcinomatosa, brochoalveoläres Karzinom und Lymphome.

Bevorzugt ist ferner die Verwendung der erfindungsgemäßen Arzneimittelformulierung zur Herstellung eines Arzneimittels zur Behandlung von Interstitiellen Lungenerkrankungen, die ausgewählt sind aus der Gruppe bestehend aus infektiös bedingte Pneumonien, wie beispielsweise aufgrund einer Infektion mit Viren, Bakterien, Pilzen, Protozoen, Helminthen oder anderen Erregern, Pneumonitis aufgrund unterschiedlicher Genese, wie beispielsweise Aspiration und Linksherzinsuffizienz, Strahlen-induzierte Pneumonitis oder Fibrose, Kollagenosen, wie beispielsweise Lupus erythematodes, systemische Sklerodermie oder Sarkoidose, Granulomatosen, wie beispielsweise Morbus Boeck, idiopathische interstitielle Pneumonie oder idiopathische pulmonäre Fibrose (IPF).

Bevorzugt ist ferner die Verwendung der erfindungsgemäßen Arzneimittelformulierung zur Herstellung eines Arzneimittels zur Behandlung von Zystischer Fibrose bzw. Mukoviszidose. Bevorzugt ist ferner die Verwendung der erfindungsgemäßen Arzneimittelformulierung zur Herstellung eines Arzneimittels zur Behandlung von Bronchitiden, wie beispielsweise Bronchitis aufgrund bakterieller oder viraler Infektion, Allergische Bronchitis und Toxische Bronchitis.

Bevorzugt ist ferner die Verwendung der erfindungsgemäßen Arzneimittelformulierung zur Herstellung eines Arzneimittels zur Behandlung von Bronchiektasen.

Bevorzugt ist ferner die Verwendung der erfindungsgemäßen Arzneimittelformulierung zur Herstellung eines Arzneimittels zur Behandlung von ARDS (adult respiratory distress syndrom).

Bevorzugt ist ferner die Verwendung der erfindungsgemäßen Arzneimittelformulierung zur Herstellung eines Arzneimittels zur Behandlung von Lungenödemen, beispielsweise toxischer Lungenödeme nach Aspiration oder Inhalation von toxischen Substanzen und Fremdstoffen.

Besonders bevorzugt betrifft die vorliegende Erfindung die Verwendung der erfindungsgemäßen Arzneimittelformulierung zur Herstellung eines Arzneimittels zur Behandlung von Asthma oder COPD. Von besonderer Bedeutung ist ferner die vorstehend genannte Verwendung zur Herstellung eines Arzneimittels zur einmal täglichen Behandlung von entzündlichen und obstruktiven Atemwegserkrankungen, besonders bevorzugt zur einmal täglichen Behandlung von Asthma oder COPD.

Ferner betrifft die vorliegende Erfindung eine Arzneimittelformulierung zur Behandlung der vorstehend genannten Erkrankungen, dadurch gekennzeichnet, dass die vorstehend genannte erfindungsgemäße Arzneimittelformulierung in therapeutisch wirksamen Mengen appliziert wird.

Die vorliegende Erfindung beschäftigt sich mit einer inhalativ applizierbaren, flüssigen Wirkstoffformulierung der Verbindung **1**, wobei die erfindungsgemäße, flüssige Formulierung hohen Qualitätsstandards genügen muss. Die erfindungsgemäße Formulierung kann dabei peroral oder pernasal inhaliert werden. Um eine optimale Wirkstoffverteilung der Wirksubstanzen in der Lunge zu erhalten, bietet sich die Applikation einer flüssigen, auf Treibgase verzichtenden, Formulierung mittels dafür geeigneter Inhalatoren an. Die inhalative Applikation einer solchen Formulierung kann sowohl auf oralem als auch auf nasalem Weg erfolgen. Besonders geeignet sind solche Inhalatoren, die eine kleine Menge einer flüssigen Formulierung in der therapeutisch notwendigen Dosierung binnen weniger Sekunden in ein therapeutisch-inhalativ geeignetes Aerosol vernebeln können. Im Rahmen der vorliegenden Erfindung sind solche Vernebler bevorzugt, bei denen bereits eine Menge von weniger als 100 Mikrolitern, bevorzugt weniger als 50 Mikrolitern, ganz bevorzugt weniger als 25 Mikrolitern Wirkstofflösung mit bevorzugt einem Hub oder zwei Hüben zu einem Aerosol mit einer durchschnittlichen Teilchengröße (bzw. Teilchendurchmesser) von weniger als 20 Mikrometern, bevorzugt weniger als 10 Mikrometern, so vernebelt werden können, dass der inhalierbare Anteil des Aerosols bereits der therapeutisch wirksamen Menge entspricht.

Eine derartige Vorrichtung zur treibgasfreien Verabreichung einer dosierten Menge eines flüssigen Arzneimittels zur inhalativen Anwendung, wird beispielsweise in der internationalen Patentanmeldung WO 91/14468 "Atomizing Device and Methods" als auch in der WO 97/12687, dort Figuren 6a und 6b und der dazugehörigen Beschreibung, ausführlich beschrieben. In einem solchen Vernebler wird eine Arzneimittellösung mittels hohen Drucks von bis zu 500 bar in ein lungengängiges Aerosol überführt und versprüht. Auf die genannten Referenzen wird im Rahmen der vorliegenden Erfindungsbeschreibung ausdrücklich in Gänze Bezug genommen.

In solchen Inhalatoren werden die Lösungsformulierungen in einem Reservoir gelagert. Dabei ist es notwendig, dass die verwendeten Wirkstoffformulierungen eine ausreichende Lagerstabilität aufweisen und gleichzeitig so beschaffen sind, dass sie dem medizinischen Zweck entsprechend möglichst ohne weitere Manipulation, direkt appliziert werden können. Ferner dürfen sie keine Bestandteile aufweisen, die so mit dem Inhalator wechselwirken können, dass der Inhalator oder die pharmazeutische Qualität der Lösung, respektive des erzeugten Aerosols, Schaden nehmen könnte.

Zur Vernebelung der Lösung wird eine spezielle Düse verwendet, wie sie beispielsweise die WO 94/07607 oder die WO 99/16530 beschreibt. Auf beide wird hiermit ausdrücklich Bezug genommen.

Es ist Aufgabe der vorliegenden Erfindung, eine wässrige, ethanolische oder wässerigethanolische Formulierung der Verbindung der Formel **1** bereitzustellen, welche den hohen Standards genügt, die notwendig sind, um eine Lösung mittels der eingangs genannten Inhalatoren optimal vernebeln zu können. Die erfindungsgemäße Wirkstoffformulierung muss dabei auch eine ausreichende pharmazeutische Qualität aufweisen, d.h. sie sollten über eine Lagerzeit von einigen Jahren, bevorzugt von mindestens einem Jahr, stärker bevorzugt von zwei Jahren pharmazeutisch stabil sein.

Diese treibgasfreien Lösungsformulierungen muss ferner mittels eines Inhalators unter Druck vernebelt werden können, wobei die im generierten Aerosol ausgebrachte Masse reproduzierbar innerhalb eines definierten Bereichs liegt.

Bezugnahmen auf die Verbindung der Formel **1** schließen im Rahmen der vorliegenden Erfindung stets alle möglichen amorphen und kristallinen Modifikationen dieser Verbindung mit ein. Bezugnahmen auf die Verbindung der Formel **1** schließen im Rahmen der vorliegenden Erfindung ferner alle möglichen Solvate und Hydrate, die von dieser Verbindung gebildet werden können mit ein.

Eine gegebenenfalls im Rahmen der vorliegenden Erfindung erfolgende Bezugnahme auf die Verbindung **1'** ist als Bezugnahme auf die in den Salzen **1** enthaltene pharmakologisch aktive freie Base der nachstehenden Formel anzusehen

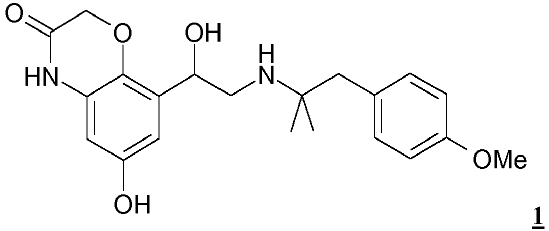

Die Konzentration der Verbindung der Formel **1** bezogen auf den Anteil an pharmakologisch wirksamer freien Base **1'** in der erfindungsgemäßen Arzneimittelformulierung liegt erfindungsgemäß bei etwa 0,75 bis 200 mg pro 100 ml. Besonders bevorzugt enthalten 100ml der erfindungsgemäßen Formulierungen etwa 1 bis etwa 100 mg **1'**.

Der pH-Wert der erfindungsgemäßen Formulierung liegt erfindungsgemäß bevorzugt in einem Bereich von 2,0 und 6,5, bevorzugt zwischen 2,2 und 5,0, besonders bevorzugt zwischen etwa 3,0 und 4,5.

Der pH-Wert wird durch Zugabe von pharmakologisch verträglichen Säuren eingestellt. Hierzu können pharmakologisch verträgliche anorganische oder organische Säuren zur Anwendung gelangen. Beispiele für bevorzugte anorganische Säuren sind ausgewählt aus der Gruppe bestehend aus Salzsäure, Bromwasserstoffsäure, Salpetersäure, Schwefelsäure und Phosphorsäure.

Beispiele für besonders geeignete organische Säuren sind ausgewählt aus der Gruppe bestehend aus Ascorbinsäure, Zitronensäure, Äpfelsäure, Weinsäure, Maleinsäure, Bernsteinsäure, Fumarsäure, Essigsäure, Ameisensäure und Propionsäure. Bevorzugte anorganische Säuren sind Salzsäure und Schwefelsäure, wobei der Salzsäure erfindungsgemäß besondere Bedeutung zukommt. Unter den organischen Säuren sind Ascorbinsäure, Fumarsäure und Zitronensäure bevorzugt, wobei Zitronensäure erfindungsgemäß besonders bevorzugt ist. Gegebenenfalls können auch Gemische der genannten Säuren eingesetzt werden, insbesondere in Fällen von Säuren, die neben ihren Säuerungseigenschaften auch andere Eigenschaften, z.B. als Geschmackstoffe oder Antioxidantien besitzen, wie beispielsweise Zitronensäure oder Ascorbinsäure. Gegebenenfalls können auch pharmakologisch verträgliche Basen zum genauen Austitrieren des pH-Wertes eingesetzt werden. Als Basen eignen sich beispielsweise Alkalihydroxide und Alkalicarbonate. Bevorzugtes Alkaliion ist Natrium. Werden solche Basen verwendet, ist darauf zu achten, dass auch die daraus resultierenden Salze, die dann in der fertigen Arzneimittelformulierung enthalten sind, mit der oben genannten Säure pharmakologisch akzeptabel sind.

Die erfindungsgemäße Formulierung kann als weitere Bestandteile Komplexbildner enthalten. Unter Komplexbildner werden im Rahmen der vorliegenden Erfindung Moleküle verstanden, die in der Lage sind Komplexbindungen einzugehen. Bevorzugt sollen durch diese Verbindungen Kationen, besonders bevorzugt metallische Kationen komplexiert werden. Die erfindungsgemäßen Formulierungen enthalten als Komplexbildner bevorzugt Editinsäure (EDTA) oder ein bekanntes Salze davon, z.B. Natrium-EDTA, bzw. Dinatrium-EDTA. Bevorzugt wird Dinatriumedetat, gegebenenfalls in Form seiner Hydrate, besonders bevorzugt in Form seines Dihydrats eingesetzt. Werden im Rahmen der erfindungsgemäßen Formulierungen Komplexbildner verwendet, so liegt deren Gehalt bevorzugt in einem Bereich von 1 bis 50 mg pro 100 ml, besonders bevorzugt in einem Bereich von 2 bis 15 mg pro 100 ml der erfindungsgemäßen Formulierung. Vorzugsweise enthalten die erfindungsgemäßen Formulierungen einen Komplexbildner in einer Menge von etwa 4 bis 12 mg pro 100 ml, besonders bevorzugt von etwa 10 mg pro 100 ml der erfindungsgemäßen Formulierung.

Analoges wie bereits für Dinatriumedetat ausgeführt, gilt auch für mögliche, mit EDTA oder seinen Salzen vergleichbare Zusatzstoffe, die komplexbildende Eigenschaften aufweisen und anstelle dessen verwendet werden können, wie beispielsweise Nitrilotriessigsäure und deren Salze.

Der erfindungsgemäßen Formulierung können weitere pharmakologisch verträgliche Hilfsstoffe zugesetzt werden. Unter Hilfs- und Zusatzstoffen wird in diesem Zusammenhang jeder pharmakologisch verträgliche und therapeutisch sinnvolle Stoff verstanden, der kein Wirkstoff ist, aber zusammen mit dem Wirkstoff in dem pharmakologisch geeigneten Lösungsmittel formuliert werden kann, um die qualitativen Eigenschaften der Wirkstoffformulierung zu verbessern. Bevorzugt entfalten diese Stoffe keine oder im Kontext mit der angestrebten Therapie keine nennenswerte oder zumindest keine unerwünschte pharmakologische Wirkung. Zu den Hilfs- und Zusatzstoffen zählen z.B. Stabilisatoren, Antioxidantien und/oder Konservierungsstoffe, die die Verwendungsdauer der fertigen Arzneimittelformulierung verlängern sowie Geschmackstoffe, Vitamine und/oder sonstige dem Stand der Technik bekannte Zusatzstoffe. Zu den Zusatzstoffen zählen auch pharmakologisch unbedenkliche Salze wie beispielsweise Natriumchlorid.

Zu den bevorzugten Hilfsstoffen zählen Antioxidantien, wie beispielsweise Ascorbinsäure, sofern nicht bereits für die Einstellung des pH-Werts verwendet, Vitamin A, Vitamin E, Tocopherole und ähnliche im menschlichen Organismus vorkommende Vitamine oder Provitamine.

Konservierungsstoffe können eingesetzt werden, um die Formulierung vor Kontamination mit pathogenen Keimen zu schützen. Als Konservierungsstoffe eignen sich die dem Stand der Technik bekannten, insbesondere Benzalkoniumchlorid oder Benzoesäure bzw. Benzoate wie Natriumbenzoat in den aus dem Stand der Technik bekannten Konzentrationen. Bevorzugt wird der erfindungsgemäßen Formulierung Benzalkoniumchlorid beigemischt. Die Menge des Benzalkoniumchlorids beträgt dabei zwischen 1 mg und 50 mg pro 100 ml Formulierung, bevorzugt etwa 2 bis 15 mg pro 100 ml, besonders bevorzugt etwa 3 bis 12 mg pro 100 ml, besonders bevorzugt etwa 4 bis 10 mg pro 100 ml der erfindungsgemäßen Formulierung. Benzalkoniumchlorid kann erfindungsgemäß auch im Gemisch mit anderen Konservierungsstoffen zum Einsatz gelangen.

Eine bevorzugte Formulierung enthält außer dem Lösungsmittel Wasser und den Verbindungen der Formel **1** nur noch Benzalkoniumchlorid, Natriumedetat und die zum Einstellen des pH-Werts notwendige Säure.

Die erfindungsgemäße Arzneimittelformulierung der Verbindung der Formel **1** wird bevorzugt in einem Inhalator der vorstehend beschriebenen Art verwendet, um daraus die erfindungsgemäßen treibgasfreien Aerosole herzustellen. An dieser Stelle sei deshalb noch einmal ausdrücklich auf die eingangs beschriebenen Patentdokumente verwiesen, auf die hiermit vollinhaltlich Bezug genommen wird.

Wie eingangs geschildert wird eine weiterentwickelte Ausführungsform des bevorzugten Inhalators in der WO 97/12687 (siehe dort insbesondere Figuren 6a und 6b und die diesbezüglichen Beschreibungsteile) offenbart. Dieser Vernebler (Respimat^{®}) kann vorteilhaft zur Erzeugung der erfindungsgemäßen inhalierbaren Aerosole eingesetzt werden. Aufgrund seiner zylinderähnlichen Form und einer handlichen Größe von weniger als 9 bis 15 cm in der Länge und 2 bis 4 cm in der Breite kann dieses Device jederzeit vom Patienten mitgeführt werden. Der Vernebler versprüht ein definiertes Volumen der Arzneimittelformulierung unter Anwendung hoher Drücke durch kleine Düsen, so dass inhalierbare Aerosole entstehen.

Im Wesentlichen besteht der bevorzugte Zerstäuber aus einem Gehäuseoberteil, einem Pumpengehäuse, einer Düse, einem Sperrspannwerk, einem Federgehäuse, einer Feder und einem Vorratsbehälter, gekennzeichnet durch
- ein Pumpengehäuse, das im Gehäuseoberteil befestigt ist, und das an seinem einen Ende einen Düsenkörper mit der Düse bzw. Düsenanordnung trägt,
- einen Hohlkolben mit Ventilkörper,
- einen Abtriebsflansch, in dem der Hohlkolben befestigt ist, und der sich im Gehäuseoberteil befindet,
- ein Sperrspannwerk, das sich im Gehäuseoberteil befindet,
- ein Federgehäuse mit der darin befindlichen Feder, das am Gehäuseoberteil mittels eines Drehlagers drehbar gelagert ist,
- ein Gehäuseunterteil, das auf das Federgehäuse in axialer Richtung aufgesteckt ist.

Der Hohlkolben mit Ventilkörper entspricht einer in der WO 97/12687 offenbarten Vorrichtungen. Er ragt teilweise in den Zylinder des Pumpengehäuses hinein und ist im Zylinder axial verschiebbar angeordnet. Insbesondere wird auf die Figuren 1-4 - insbesondere Figur 3 - und die dazugehörigen Beschreibungsteile der o.g. Internationalen Patentanmeldung Bezug genommen. Der Hohlkolben mit Ventilkörper übt auf seiner Hochdruckseite zum Zeitpunkt des Auslösens der Feder einen Druck von 5 bis 60 MPa (etwa 50 bis 600 bar), bevorzugt 10 bis 60 MPa (etwa 100 bis 600 bar) auf das Fluid, die abgemessene Wirkstofflösung aus. Dabei werden Volumina von 10 bis 50 Mikroliter bevorzugt, besonders bevorzugt sind Volumina von 10 bis 20 Mikroliter, ganz besonders bevorzugt ist ein Volumen von 10 bis 15 Mikroliter pro Hub.

Der Ventilkörper ist bevorzugt an dem Ende des Hohlkolbens angebracht, das dem Düsenkörper zugewandt ist.

Die Düse im Düsenkörper ist bevorzugt mikrostrukturiert, d.h. durch Mikrotechnik hergestellt. Mikrostrukturierte Düsenkörper sind beispielsweise in der WO-99/16530 offenbart; auf diese Schrift wird hiermit inhaltlich Bezug genommen, insbesondere auf die dort offenbarte Figur 1 und deren Beschreibung. Der Düsenkörper besteht z.B. aus zwei fest miteinander verbundenen Platten aus Glas und/oder Silizium, von denen wenigstens eine Platte einen oder mehrere mikrostrukturierte Kanäle aufweist, die die Düseneinlaßseite mit der Düsenauslaßseite verbinden. Auf der Düsenauslaßseite ist mindestens eine runde oder nicht-runde Öffnung von 2 bis 10 Mikrometer Tiefe und 5 bis 15 Mikrometern Breite, wobei die Tiefe bevorzugt bei 4, 5 bis 6,5 Mikrometern und die Länge bei 7 bis 9 Mikrometern beträgt. Im Fall von mehreren Düsenöffnungen, bevorzugt sind zwei, können die Strahlrichtungen der Düsen im Düsenkörper parallel zueinander verlaufen oder sie sind in Richtung Düsenöffnung gegeneinander geneigt. Bei einem Düsenkörper mit mindestens zwei Düsenöffnungen auf der Auslaßseite können die Strahlrichtungen mit einem Winkel von 20 Grad bis 160 Grad gegeneinander geneigt sein, bevorzugt wird ein Winkel von 60 bis 150 Grad, insbesondere bevorzugt 80 bis 100°. Die Düsenöffnungen sind bevorzugt in einer Entfernung von 10 bis 200 Mikrometern angeordnet, stärker bevorzugt in einer Entfernung von 10 bis 100 Mikrometer, besonders bevorzugt 30 bis 70 Mikrometer. Am stärksten bevorzugt sind 50 Mikrometer.
Die Strahlrichtungen treffen sich dementsprechend in der Umgebung der Düsenöffnungen.

Die flüssige Arzneimittelformulierung trifft wie bereits erwähnt mit einem Eingangsdruck von bis zu 600 bar, bevorzugt 200 bis 300 bar auf den Düsenkörper und wird über die Düsenöffnungen in ein inhalierbares Aerosol zerstäubt. Die bevorzugten Teilchengrößen des Aerosols liegen bei bis zu 20 Mikrometern, bevorzugt 3 bis 10 Mikrometern.

Das Sperrspannwerk enthält eine Feder, bevorzugt eine zylindrische schraubenförmige Druckfeder, als Speicher für die mechanische Energie. Die Feder wirkt auf den Abtriebsflansch als Sprungstück, dessen Bewegung durch die Position eines Sperrglieds bestimmt wird. Der Weg des Abtriebsflansches wird durch einen oberen und einen unteren Anschlag präzise begrenzt. Die Feder wird bevorzugt über ein kraftübersetzendes Getriebe, z.B. ein Schraubschubgetriebe, durch ein äußeres Drehmoment gespannt, das beim Drehen des Gehäuseoberteils gegen das Federgehäuse im Gehäuseunterteil erzeugt wird. In diesem Fall enthalten das Gehäuseoberteil und der Abtriebsflansch ein ein- oder mehrgängiges Keilgetriebe.

Das Sperrglied mit einrückenden Sperrflächen ist ringförmig um den Abtriebsflansch angeordnet. Es besteht z.B. aus einem in sich radial elastisch verformbaren Ring aus Kunststoff oder aus Metall. Der Ring ist in einer Ebene senkrecht zur Zerstäuberachse angeordnet. Nach dem Spannen der Feder schieben sich die Sperrflächen des Sperrgliedes in den Weg des Abtriebsflansches und verhindern das Entspannen der Feder. Das Sperrglied wird mittels einer Taste ausgelöst. Die Auslösetaste ist mit dem Sperrglied verbunden oder gekoppelt. Zum Auslösen des Sperrspannwerkes wird die Auslösetaste parallel zur Ringebene, und zwar bevorzugt in den Zerstäuber hinein, verschoben; dabei wird der verformbare Ring in der Ringebene verformt. Konstruktive Details des Sperrspannwerkes sind in der WO 97/20590 beschrieben.

Das Gehäuseunterteil wird in axialer Richtung über das Federgehäuse geschoben und verdeckt die Lagerung, den Antrieb der Spindel und den Vorratsbehälter für das Fluid.

Beim Betätigen des Zerstäubers wird das Gehäuseoberteil gegen das Gehäuseunterteil gedreht, wobei das Gehäuseunterteil das Federgehäuse mitnimmt. Dabei wird die Feder über das Schraubschubgetriebe zusammengedrückt und gespannt, und das Sperrwerk rastet selbsttätig ein. Der Drehwinkel ist bevorzugt ein ganzzahliger Bruchteil von 360 Grad, z.B. 180 Grad. Gleichzeitig mit dem Spannen der Feder wird das Abtriebsteil im Gehäuseoberteil um einen vorgegebenen Weg verschoben, der Hohlkolben wird innerhalb des Zylinders im Pumpengehäuse zurückgezogen, wodurch eine Teilmenge des Fluids aus dem Vorratsbehälter in den Hochdruckraum vor der Düse eingesaugt wird.

In den Zerstäuber können gegebenenfalls nacheinander mehrere das zu zerstäubende Fluid enthaltende austauschbare Vorratsbehälter eingeschoben und benutzt werden. Der Vorratsbehälter enthält die erfindungsgemäße Aerosolzubereitung.

Der Zerstäubungsvorgang wird durch leichtes Eindrücken der Auslösetaste eingeleitet. Dabei gibt das Sperrwerk den Weg für das Abtriebsteil frei. Die gespannte Feder schiebt den Kolben in den Zylinder des Pumpengehäuses hinein. Das Fluid tritt aus der Düse des Zerstäubers in zerstäubter Form aus.

Weitere konstruktive Details sind in den PCT-Anmeldungen WO 97/12683 und WO 97/20590 offenbart, auf die hiermit inhaltlich Bezug genommen wird.

Die Bauteile des Zerstäubers (Vernebiers) sind aus einem der Funktion entsprechend geeignetem Material. Das Gehäuse des Zerstäubers und - so weit es die Funktion erlaubt - auch andere Teile sind bevorzugt aus Kunststoff, z.B. im Spritzgießverfahren, hergestellt. Für medizinische Zwecke werden physiologisch unbedenkliche Materialien verwendet.

In den Figuren 6 a/b der WO 97/12687, ist der Vernebler (Respimat®) beschrieben, mit dem die erfindungsgemäßen wässrigen Aerosolzubereitungen vorteilhaft inhaliert werden können. Figur 6 a zeigt einen Längsschnitt durch den Zerstäuber bei gespannter Feder, Figur 6 b zeigt einen Längsschnitt durch den Zerstäuber bei entspannter Feder.

Das Gehäuseoberteil (51) enthält das Pumpengehäuse (52), an dessen Ende der Halter (53) für die Zerstäuberdüse angebracht ist. In dem Halter befindet sich der Düsenkörper (54) und ein Filter (55). Der im Abtriebsflansch (56) des Sperrspannwerkes befestigte Hohlkolben (57) ragt teilweise in den Zylinder des Pumpengehäuses hinein. An seinem Ende trägt der Hohlkolben den Ventilkörper (58). Der Hohlkolben ist mittels der Dichtung (59) abgedichtet. Innerhalb des Gehäuseoberteils befindet sich der Anschlag (60), an dem der Abtriebsflansch bei entspannter Feder anliegt. Am Abtriebsflansch befindet sich der Anschlag (61), an dem der Abtriebsflansch bei gespannter Feder anliegt. Nach dem Spannen der Feder schiebt sich das Sperrglied (62) zwischen den Anschlag (61) und eine Abstützung (63) im Gehäuseoberteil. Die Auslösetaste (64) steht mit dem Sperrglied in Verbindung. Das Gehäuseoberteil endet im Mundstück (65) und ist mit der aufsteckbaren Schutzkappe (66) verschlossen.

Das Federgehäuse (67) mit Druckfeder (68) ist mittels der Schnappnasen (69) und Drehlager am Gehäuseoberteil drehbar gelagert. Über das Federgehäuse ist das Gehäuseunterteil (70) geschoben. Innerhalb des Federgehäuses befindet sich der austauschbare Vorratsbehälter (71) für das zu zerstäubende Fluid (72). Der Vorratsbehälter ist mit dem Stopfen (73) verschlossen, durch den der Hohlkolben in den Vorratsbehälter hineinragt und mit seinem Ende in das Fluid (Vorrat an Wirkstofflösung) eintaucht.

In der Mantelfläche des Federgehäuses ist die Spindel (74) für das mechanische Zählwerk angebracht. An dem Ende der Spindel, das dem Gehäuseoberteil zugewandt ist, befindet das Antriebsritzel (75). Auf der Spindel sitzt der Reiter (76).

Der oben beschriebene Vernebler ist geeignet, die erfindungsgemäßen Aerosolzubereitungen zu einem für die Inhalation geeignetem Aerosol zu vernebeln.

Wird die erfindungsgemäße Formulierung mittels der vorstehend beschriebenen Technik (Respimat^{®}) vernebelt, sollte die ausgebrachte Masse bei wenigstens 97%, bevorzugt wenigstens 98% aller Betätigungen des Inhalators (Hub oder Hübe) einer definierten Menge mit einem Toleranzbereichs von maximal 25%, bevorzugt 20% dieser Menge entsprechen. Bevorzugt werden pro Hub zwischen 5 und 30 mg Formulierung als definierte Masse ausgebracht, besonders bevorzugt zwischen 5 und 20 mg.

Die erfindungsgemäße Formulierung kann auch mittels anderer als der vorstehend beschriebenen Inhalatoren, beispielsweise Jet-Stream-Inhalatoren, vernebelt werden.

Die vorliegende Erfindung betrifft ferner ein Inhalationskit bestehend aus einer der vorstehend beschriebenen erfindungsgemäßen Arzneimittelzubereitungen und einem zur Vernebelung dieser Arzneimittelformulierung geeigneten Inhalator.

Die vorliegende Erfindung betrifft bevorzugt ein Inhalationskit bestehend aus einer der vorstehend beschriebenen erfindungsgemäßen Arzneimittelzubereitungen und dem vorstehend beschriebenen Inhalator Respimat^{®}.

### EXPERIMENTELLER TEIL

Die nachstehend ausgeführten Formulierungsbeispiele dienen der weitergehenden Erläuterung.

### I. Darstellung der Verbindungen der Formel 1: 6-Hydroxy-8-{1-hydroxy-2-[2-(4-methoxy-phenyl)-1,1-dimethyl-ethylamino]-ethyl}-4H-benzo[1,4]oxazin-3-on-Hydrochlorid

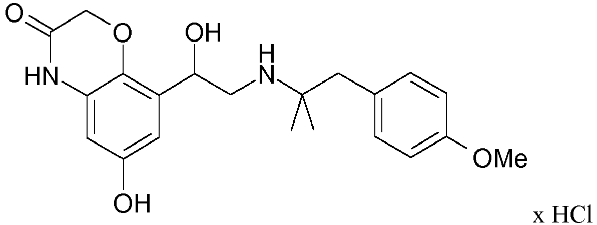

**a) 8-{2-[1,1-Dimethyl-2-(4-methoxy-phenyl)-ethylamino]-1-hydroxy-ethyl}-6-benzyloxy-4H-benzo[1,4]oxazin-3-on:** Zu einer Lösung von 3.6 g 1,1-Dimethyl-2-(4-methoxyphenyl)-ethylamin in 100 mL Ethanol werden bei 70°C 7.5 g (6-Benzyloxy-4H-benzo[1,4]oxazin-3-on)-glyoxalhydrat gegeben und man lässt 15 Minuten rühren. Anschließend werden innerhalb von 30 Minuten bei 10 bis 20°C 1 g Natriumborhydrid zugesetzt. Es wird eine Stunde gerührt, mit 10 mL Aceton versetzt und über weitere 30 Minuten gerührt. Die Reaktionsmischung wird mit 150 mL Ethylacetat verdünnt, mit Wasser gewaschen, mit Natriumsulfat getrocknet und eingeengt. Der Rückstand wird in 50 mL Methanol und 100 mL Ethylacetat gelöst und mit konz. Salzsäure sauer gestellt. Nach Zugabe von 100 mL Diethylether fällt das Produkt aus. Die Kristalle werden abfiltriert, gewaschen und in 50 mL Ethanol umkristallisiert. Ausbeute: 7 g (68%; Hydrochlorid); Schmp. = 232-234°C.
**b) 8-{2-[1,1-Dimethyl-2-(4-methoxy-phenyl)-ethylamino]-1-hydroxy-ethyl}-6-hydroxy-4H-benzo[1,4]oxazin-3-on-Hydrochlorid:** 6.8 g der vorstehend erhaltenen Benzylverbindung werden in 125 mL Methanol unter Zusatz von 1 g Palladium auf Kohle (5%ig) bei Raumtemperatur und Normaldruck hydriert. Der Katalysator wird abfiltriert und das Filtrat vom Lösungsmittel befreit. Nach Umkristallisation des Rückstandes in 50 mL Aceton und etwas Wasser wird ein Feststoff erhalten, der abfiltriert und gewaschen wird. Ausbeute: 5.0 g (89 %; Hydrochlorid); Schmp. = 155-160°C.

Die (R)- und (S)-Enantiomere von Beispiel 3 können aus dem Racemat beispielsweise mittels chiraler HPLC erhalten werden (z.B. Säule: Chirobiotic T, 250 x 22.1 mm von der Firma Astec). Als mobile Phase kann Methanol mit 0.05 % Triethylamin und 0.05% Essigsäure verwendet werden. Kieselgel mit einer Korngröße von 5 µm, an das kovalent das Glykoprotein Teicoplanin gebunden ist, kann als Säulenmaterial zum Einsatz gelangen. Retentionszeit (R-Enantiomer) = 40.1 min, Retentionszeit (S-Enantiomer) = 45.9 min. Die beiden Enantiomere werden nach dieser Methode in Form der freien Basen erhalten und sind durch Umsetzung mit der gewünschten Säure (z.B. Salzsäure) nach im Stand der Technik allgemein bekannten Vorgehensweisen in die entsprechenden Säureadditionssalze überführbar.

Erfindungsgemäß von herausragender Bedeutung ist das R-Enantiomer des Beispiels 3.

### II. Formulierungsbeispiele

In der nachstehenden Tabelle sind erfindungsgemäße Formulierungsbeispiele des R-Enantiomers der Verbindung Beispiel 3 zusammengefasst. 100 ml Arzneimittelformulierung enthalten in gereinigtem Wasser bzw. Wasser für Injektionszwecke:

| Beispiel | **1** (**1'**-HCl) (mg) | Benzalkoniumchlorid (mg) | Dinatriumedetat-Dihydrat (mg) | Zitronensäure (mg) |
|---|---|---|---|---|
| 1 | 10 | 10 | - | 3 |
| 2 | 1,0 | 15 | - | 5 |
| 3 | 100 | - | - | 5 |
| 4 | 10 | - | 5 | 3 |
| 5 | 1,0 | - | 10 | 3 |
| 6 | 0,5 | 5 | 7 | 2 |
| 7 | 1000 | 5 | 15 | 4 |
| 8 | 90 | 10 | 10 | 3 |
| 9 | 23 | 10 | 10 | 3 |
| 10 | 2,7 | 10 | 10 | 3 |
| 11 | 0,5 | 15 | 10 | 2 |

In der nachstehenden Tabelle sind erfindungsgemäße Formulierungsbeispiele des R-Enantiomers der Verbindung Beispiel 3 zusammengefasst. 100 ml Arzneimittelzubereitung enthalten:

| Beispiel | **1** (**1'**-HCl) (mg) | Benzalkoniumchlorid (mg) | Dinatriumedetat-Dihydrat (mg) | Zitronensäure (mg) | Auf 100 ml mit Ethanol/Wasser Mischung (% m/m) |
|---|---|---|---|---|---|
| 1 | 10 | 10 | 10 | 3 | 20/80 |
| 2 | 10 | 10 | 10 | 3 | 50/50 |
| 3 | 1,0 | 5 | - | 3 | 70/30 |
| 4 | 100 | - | 10 | 5 | 70/30 |
| 5 | 10 | - | 20 | 2 | 70/30 |
| 6 | 1,0 | - | 10 | 3 | 90/10 |
| 7 | 0,5 | - | 10 | 2 | 90/10 |
| 8 | 1000 | - | - | 4 | 90/10 |
| 9 | 100 | - | - | 3 | 90/10 |
| 10 | 10 | - | - | 4 | 95/5 |
| 11 | 2,5 | - | - | 3 | 95/5 |
| 12 | 0,5 | 5 | - | 3 | 95/5 |
| 13 | 10 | - | 5 | 3 | 100/0 |
| 14 | 10 | 5 | - | 3 | 100/0 |

## Patentansprüche

1. Arzneimittelformulierung, enthaltend als alleinigen Wirkstoff eine Verbindung der allgemeinen Formel **1** 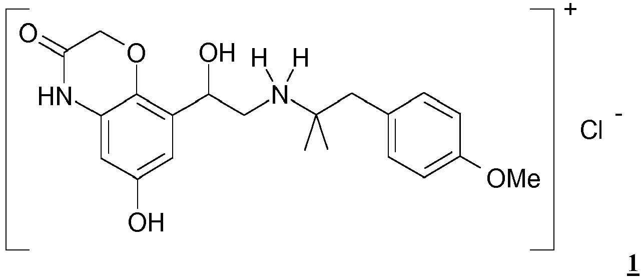 gegebenenfalls in Form Ihrer Tautomere, Enantiomere, Mischungen der Enantiomere, Racemate oder Solvate, wenigstens eine pharmakologisch verträgliche Säure, gegebenenfalls weitere pharmakologisch verträgliche Hilfsstoffe und/oder Komplexbildner sowie als Lösungsmittel Wasser, Ethanol oder ein Gemisch aus Wasser und Ethanol,
wobei die Konzentration der Verbindung der Formel **1** bezogen auf den Anteil an pharmakologisch wirksamer freien Base **1'** bei 0,75 bis 200 mg pro 100 ml liegt.

2. Arzneimittelformulierung nach Anspruch 1, worin die pharmakologisch verträgliche Säure ausgewählt ist aus den anorganischen Säuren Salzsäure, Bromwasserstoffsäure, Salpetersäure, Schwefelsäure und Phosphorsäure oder aus den organischen Säuren Ascorbinsäure, Zitronensäure, Äpfelsäure, Weinsäure, Maleinsäure, Bernsteinsäure, Fumarsäure, Essigsäure, Ameisensäure und Propionsäure.

3. Arzneimittelformulierung nach einem der Ansprüche 1 oder 2, **gekennzeichnet durch** einen pH von 2,5 bis 6,5.

4. Arzneimittelformulierung nach einem der Ansprüche 1 bis 3, **dadurch gekennzeichnet, dass** sie als Hilfsstoff Benzalkoniumchlorid enthalten.

5. Arzneimittelformulierung nach Anspruch 4, **dadurch gekennzeichnet dass** der Gehalt an Benzalkoniumchlorid 1 bis 50 mg pro 100 ml Lösung beträgt.

6. Arzneimittelformulierung nach einem der Ansprüche 1 bis 5, **dadurch gekennzeichnet dass** die Konzentration der Verbindung der Formel **1** bezogen auf den Anteil an pharmakologisch wirksamer freien Base **1'** bei 1 bis 100 mg pro 100 ml liegt.

7. Arzneimittelformulierung nach einem der Ansprüche 1 bis 6, **dadurch gekennzeichnet, dass** sie als weiteren Bestandteil einen Komplexbildner enthalten.

8. Arzneimittelformulierung nach Anspruch 7, **dadurch gekennzeichnet dass** der Gehalt an Komplexbildner 1 bis 50 mg pro 100 ml Lösung beträgt.

9. Arzneimittelformulierung nach einem der Ansprüche 1 bis 8, **dadurch gekennzeichnet dass** sie als Lösungsmittel reines Wasser enthalten.

10. Arzneimittelformulierung nach einem der Ansprüche 1 bis 8, **dadurch gekennzeichnet dass** sie als Lösungsmittel reines Ethanol enthalten.

11. Arzneimittelformulierung nach einem der Ansprüche 1 bis 8, **dadurch gekennzeichnet dass** sie als Lösungsmittel eine Gemisch aus Wasser und Ethanol enthalten.

12. Arzneimittelformulierung nach Anspruch 11, **dadurch gekennzeichnet dass** sie als Lösungsmittel eine Gemisch aus Wasser und Ethanol enthält, in dem der prozentuale Massenanteil von Ethanol im Bereich zwischen 5 und 99 % Ethanol liegt.

13. Arzneimittelformulierung, enthaltend als alleinigen Wirkstoff eine Verbindung der Formel ***R*-1** 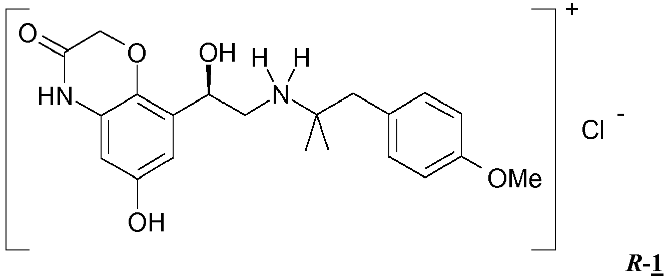 gegebenenfalls in Form Ihrer Tautomere oder Solvate, wenigstens eine pharmakologisch verträgliche Säure, gegebenenfalls weitere pharmakologisch verträgliche Hilfsstoffe und/oder Komplexbildner sowie als Lösungsmittel Wasser, Ethanol oder ein Gemisch aus Wasser und Ethanol, wobei die Konzentration der Verbindung der Formel ***R*-1** bezogen auf den Anteil an pharmakologisch wirksamer freien Base **1'** bei 0,75 bis 200 mg pro 100 ml liegt.

14. Arzneimittelformulierung (100 ml) in gereinigtem Wasser, worin 23 mg Wirkstoff eine Verbindung der Formel (***R*-1**), 10 mg Benzalkoniumchlorid, 10 mg Dinatriumedetat Dihydrat und 3 mg Zitronensäure enthaltend sind.

15. Verwendung einer Arzneimittelformulierung nach einem der Ansprüche 1 bis 14 zur Herstellung eines Arzneimittels zur Behandlung von Atemwegserkrankungen.

16. Inhalationskit bestehend aus einer Arzneimittelformulierung nach einem der Ansprüche 1 bis 14 und einem zur Vernebelung dieser Arzneimittelformulierung geeigneten Inhalator.

17. Inhalationskit nach Anspruch 16, wobei der Inhalator der Respimat^{®} ist.

## Claims

1. Medicament formulation containing as sole active substance a compound of general formula **1** 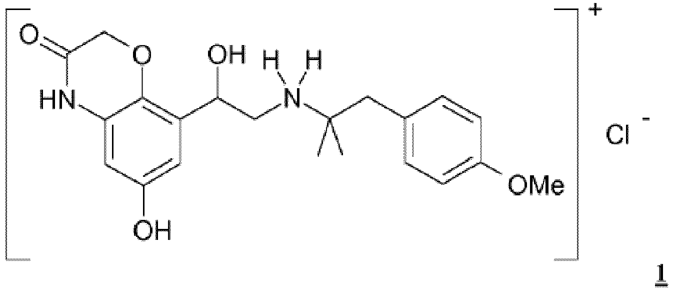 optionally in the form of its tautomers, enantiomers, mixtures of enantiomers, racemates or solvates, at least one pharmacologically acceptable acid, optionally other pharmacologically acceptable excipients and/or complexing agents and, as solvent, water, ethanol or a mixture of water and ethanol, wherein the concentration of the compound of formula **1** based on the amount of pharmacologically active free base **1'** is 0.75 to 200 mg per 100 ml.

2. Medicament formulation according to claim 1, wherein the pharmacologically acceptable acid is selected from the inorganic acids hydrochloric acid, hydrobromic acid, nitric acid, sulphuric acid and phosphoric acid or from the organic acids ascorbic acid, citric acid, malic acid, tartaric acid, maleic acid, succinic acid, fumaric acid, acetic acid, formic acid and propionic acid.

3. Medicament formulation according to one of claims 1 or 2, **characterised by** a pH of 2.5 to 6.5.

4. Medicament formulation according to one of claims 1 to 3, **characterised in that** it contains benzalkonium chloride as excipient.

5. Medicament formulation according to claim 4, **characterised in that** the content of benzalkonium chloride is 1 to 50 mg per 100 ml solution.

6. Medicament formulation according to one of claims **1** to 5, **characterised in that** the concentration of the compound of formula **1** based on the amount of pharmacologically active free base **1'** is 1 to 100 mg per 100 ml.

7. Medicament formulation according to one of claims 1 to 6, **characterised in that** it contains a complexing agent as a further ingredient.

8. Medicament formulation according to claim 7, **characterised in that** the content of complexing agent is 1 to 50 mg per 100 ml solution.

9. Medicament formulation according to one of claims 1 to 8, **characterised in that** it contains pure water as solvent.

10. Medicament formulation according to one of claims 1 to 8, **characterised in that** it contains pure ethanol as solvent.

11. Medicament formulation according to one of claims 1 to 8, **characterised in that** it contains a mixture of water and ethanol as solvent.

12. Medicament formulation according to claim 11, **characterised in that** it contains as solvent a mixture of water and ethanol, wherein the percentage proportion of ethanol by mass is in the range from 5 to 99 % ethanol.

13. Medicament formulation containing as sole active substance a compound of formula ***R*-1** 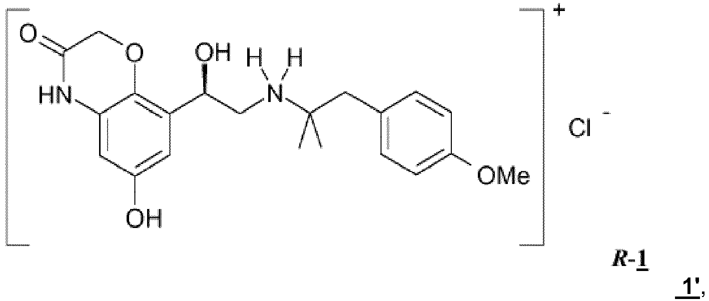 optionally in the form of its tautomers or solvates, at least one pharmacologically acceptable acid, optionally other pharmacologically acceptable excipients and/or complexing agents and, as solvent, water, ethanol or a mixture of water and ethanol, wherein the concentration of the compound of formula ***R*-1** based on the amount of pharmacologically active free base **1'** is 0.75 to 200 mg per 100 ml.

14. Medicament formulation (100 ml) in purified water, wherein 23 mg of active substance contain a compound of formula (***R*-1**), 10 mg of benzalkonium chloride, 10 mg of disodium edetate dihydrate and 3 mg of citric acid.

15. Use of a medicament formulation according to one of claims 1 to 14 for preparing a medicament for the treatment of respiratory complaints.

16. Inhalation kit consisting of a medicament formulation according to one of claims 1 to 14 and an inhaler suitable for nebulising this medicament formulation.

17. Inhalation kit according to claim 16, wherein the inhaler is a Respimat^{®}.

## Revendications

1. Formulation médicamenteuse contenant comme substance active unique un composé de formule générale 1 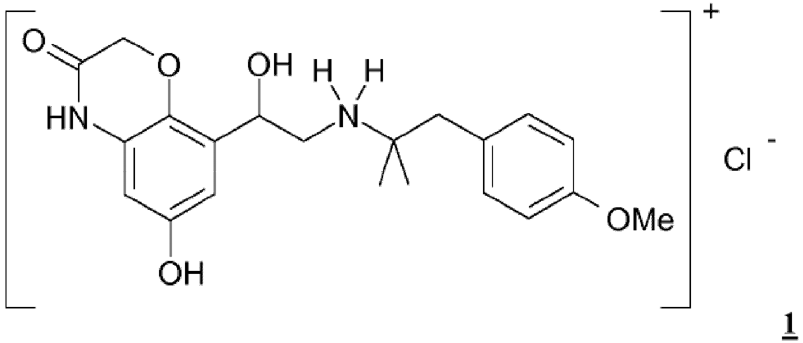 éventuellement sous la forme de ses tautomères, ses énantiomères, ses mélanges d'énantiomères, ses racémates ou ses solvates, au moins un acide pharmacologiquement acceptable, éventuellement d'autres adjuvants et/ou agents complexants pharmacologiquement acceptables et comme solvant de l'eau, de l'éthanol ou un mélange d'eau et d'éthanol, dans laquelle la concentration du composé de formule 1 par rapport à la proportion de base libre 1' pharmacologiquement efficace est de 0,75 à 200 mg pour 100 ml.

2. Formulation médicamenteuse selon la revendication 1, dans laquelle l'acide pharmacologiquement acceptable est choisi parmi les acides inorganiques acide chlorhydrique, acide bromhydrique, acide nitrique, acide sulfurique et acide phosphorique ou parmi les acides organiques acide ascorbique, acide citrique, acide malique, acide tartrique, acide maléique, acide succinique, acide fumarique, acide acétique, acide formique et acide propionique.

3. Formulation médicamenteuse selon l'une des revendications 1 ou 2, **caractérisée par** un pH de 2,5 à 6,5.

4. Formulation médicamenteuse selon l'une des revendications 1 à 3, **caractérisée en ce qu'**elle contient comme adjuvant du chlorure de benzalkonium.

5. Formulation médicamenteuse selon la revendication 4, **caractérisée en ce que** la teneur en chlorure de benzalkonium est de 1 à 50 mg pour 100 ml de solution.

6. Formulation médicamenteuse selon l'une des revendications 1 à 5, **caractérisée en ce que** la concentration du composé de formule 1 par rapport à la teneur en base libre 1' pharmacologiquement efficace est de 1 à 100 mg pour 100 ml.

7. Formulation médicamenteuse selon l'une des revendications 1 à 6, **caractérisée en ce qu'**elle contient comme autre composant un agent complexant.

8. Formulation médicamenteuse selon la revendication 7, **caractérisée en ce que** la teneur en agent complexant est de 1 à 50 mg pour 100 ml de solution.

9. Formulation médicamenteuse selon l'une des revendications 1 à 8, **caractérisée en ce qu'**elle contient comme solvant de l'eau pure.

10. Formulation médicamenteuse selon l'une des revendications 1 à 8, **caractérisée en ce qu'**elle contient comme solvant de l'éthanol pur.

11. Formulation médicamenteuse selon l'une des revendications 1 à 8, **caractérisée en ce qu'**elle contient comme solvant un mélange d'eau et d'éthanol.

12. Formulation médicamenteuse selon la revendication 11, **caractérisée en ce qu'**elle contient comme solvant un mélange d'eau et d'éthanol dans lequel le pourcentage en poids d'éthanol se situe dans une plage entre 5 et 99 %.

13. Formulation médicamenteuse, contenant comme substance active unique un composé de formule *R*-1 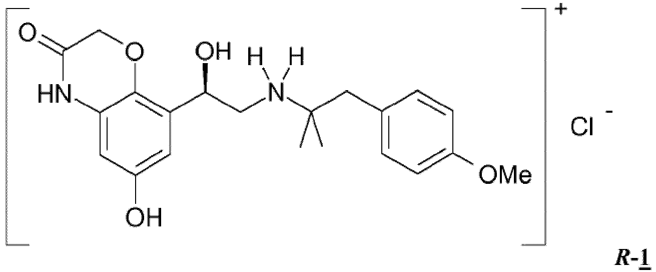 éventuellement sous la forme de ses tautomères ou ses solvates, au moins un acide pharmacologiquement acceptable, éventuellement d'autres adjuvants et/ou agents complexants pharmacologiquement acceptables et comme solvants de l'eau, de l'éthanol ou un mélange d'eau et d'éthanol, la concentration du composé de formule *R*-1 par rapport à la proportion de base libre 1' pharmacologiquement efficace étant de 0,75 à 200 mg pour 100 ml.

14. Formulation médicamenteuse (100 ml) dans de l'eau purifiée, contenant 23 mg de substance active d'un composé de formule *(R-1),* 10 mg de chlorure de benzalkonium, 10 mg de dihydrate d'édétate disodique et 3 mg d'acide citrique.

15. Utilisation d'une formulation médicamenteuse selon l'une des revendications 1 à 14, pour la production d'un médicament pour le traitement de maladies des voies respiratoires.

16. Kit d'inhalation consistant en une formulation médicamenteuse selon l'une des revendications 1 à 14 et un inhalateur approprié pour la nébulisation de cette formulation médicamenteuse.

17. Kit d'inhalation selon la revendication 16, dans lequel l'inhalateur est le Respimat®.
